# EUROPEAN PATENT APPLICATION

(11) **EP 4 501 325 A1**
(43) Date of publication of application: **05.02.2025**
(21) Application number: 22933775.3
(22) Date of filing: 15.11.2022
(51) Int. Cl.: A61K 31/423, A61P 27/02, A23L 33/10

(54) **NOVEL COMPOUND INDUCING EXPRESSION OF ANTI-AGING GENE KLOTHO**

(30) Priority: 22.03.2022 KR 20220035142
(71) Applicant: Klotho Sciences Co., Ltd., Seongnam-si, Gyeonggi-do 13605 (KR)
(72) Inventor: JUNG, Dong Ju, Cheonan-si, Chungcheongnam-do 31166 (KR)
(74) Representative: Dehns
(86) International application number: PCT/KR2022/017991
(87) International publication number: WO 2023/182606

(57) **Abstract**

The present invention relates to novel compounds that induce the expression of the anti-aging gene klotho. The compounds according to the invention are not only effective in enhancing the expression of the Klotho gene, a gene associated with aging, but also have a protective effect against oxidative stress in retinal pigment epithelial cells (RPE), and are therefore useful as a pharmaceutical composition or as a health functional food composition for the prevention or treatment of macular degeneration.

## Description

### [Technical field]

This application claims priority to Korean Patent Application No. 10-2022-0035142, filed March 22, 2023, which is hereby incorporated by reference in its entirety.

The present invention relates to novel compounds that induce the expression of the anti-aging gene KLOTHO.

### [Background art]

The fact that genes that can control aging in animals may exist became known from the senescence-accelerated mice (SAM) reported in 1981. This mouse, which was accidentally created while crossing AKR/J mice, aged much faster than mice of the same line, and it was confirmed that this mouse had mutations in several genes. Later, the discovery of a single group of aging-related genes was reported in the 1990s. The genes reported were RecQ family genes that express an enzyme called DNA helicase. Mutations in these genes have been reported to result in premature aging or cancer, which is known to occur by affecting DNA repair. The single gene associated with aging is the klotho gene, which was reported in 1997. The klotho gene was accidentally discovered while creating an animal model for transgenic hypertension mice, but in mice that could not express this gene, premature aging occurred and their lives were shortened. More interestingly, the lifespan of mice that later increased the expression of this gene increased by 20.0 to 30.8% in males and 18.8 to 19.0% in females. This became the first opportunity to inform the world that the lifespan of mice can be increased or decreased depending on the expression of a single gene. In addition, the base sequence of the klotho gene was very similar among animals, and it was reported that it was about 98% identical between mice and humans. This indicates that lifespan can be regulated according to the expression of the klotho gene in humans as well.

In humans, the klotho gene is located on chromosome 13 and produces a membrane protein that is sequence similar to β-glucosidase. Klotho protein is predominantly expressed in tubular epithelial cells of the kidney and choroid plexus of the brain, and has been reported to be expressed in some parathyroid glands. The Klotho gene has been implicated in a variety of aging phenotypes, and mice lacking the klotho gene develop aging-like syndromes, including reduced lifespan, decreased activity, growth retardation, atherosclerosis, arterial calcification, osteoporosis, genital immaturity, infertility, skin atrophy, and emphysema. In klotho mutant mice, atherosclerosis similar to Monckeberg type arteriosclerosis caused by aging in humans is observed in all arteries from the aorta to the arterioles, and angiogenesis and vasculogenesis are impaired.

Klotho mRNA is significantly higher in kidney tissue than in other tissues, but its expression is reduced in the kidneys of rats with disease models of hypertension, type 2 diabetes, diabetic nephropathy, and chronic kidney failure. In rats with reduced klotho expression, production of nitric oxide (NO), a vascular endothelium-derived relaxing factor, is reduced, and injection of the klotho gene into OLETF (Otsuka Long-Evans Tokushima fatty) rats, which have multiple risk factors for cardiovascular disease, using a viral gene delivery system, improves endothelial function, increases NO production, and reduces blood pressure by inhibiting vascular thickening and fibrosis. The klotho gene also affects glucose and insulin metabolism in rats, and statins, a common treatment for hypercholesterolemia, increase the expression of klotho mRNA in renal proximal tubule cells. Klotho knockout rats develop osteopenia with low bone turnover due to impaired differentiation of both osteoblasts and osteoclasts, which is similar to age-related bone loss and senile osteoporosis in humans. Klotho mutant rats also exhibit abnormal elongation of trabecular bone at bone ends and abnormal trabecular bone texture on microcomputed tomography, which is attributed to impaired bone resorption. The clinical phenotypic changes caused by Klotho gene mutations observed in humans are variable. The functional variant of klotho (KL-VS) modification with mutations in three sites of the klotho gene exon2 is associated with lipid metabolism, blood pressure, lifespan, cognitive function, coronary artery disease and cerebrovascular disease, and microsatellite polymorphism and single base genetic polymorphism of the klotho gene are associated with bone density, and it has also been reported that the single base genetic polymorphism of the klotho gene in healthy adult women is associated with cardiovascular disease risk factors and bone density. Recently, the Klotho gene has also been linked to Alzheimer's disease in several papers. In a mouse model of Alzheimer's disease, overexpression of klotho was reported to increase the lifespan of mice by 30% and inhibit cognitive decline. In addition, a 50% reduction in the production of amyloid beta protein in the brain was observed with klotho expression. In humans, klotho expression is inversely correlated with the progression of Alzheimer's disease, and reports have been submitted that klotho protein reduces the amounts of inflammatory cytokines in the blood of Alzheimer's patients. Several studies have also reported that Klotho enhances retinal pigment epithelium (RPE) function and protects RPE cells from oxidative stress. Retinas of Klotho gene knockout (KL-/-) mice show degeneration of photoreceptors and reduced pigment production in RPE cells. It has been shown that klotho is expressed in cultured RPE cells and that the expressed klotho protein increases the synthesis of L-3,4-dihydorxyphenylalanine (L-DOPA) and inhibits the production of vascular endothelial growth factor (VEGF) from the basement membrane. In RPE cells, klotho has also been shown to protect RPE cells from oxidative stress by reducing the production of reactive oxygen species (ROS). In patients with age-related macular degeneration (AMD), klotho expression was reduced in the aqueous humor of the eye, and this reduction was associated with oxidative stress and inflammation.

The present inventors continued to conduct research on compounds that induce klotho expression, and experimentally confirmed that the new compounds developed by the present inventors have excellent stability and have excellent protective effects against oxidative stress (oxidotoxicity) in retinal pigment epithelial cells (RPE), and completed the present invention.

### [Disclosure]

### [Technical Problem]

It is an object of the present invention to provide novel compounds represented by the following formula 1 and pharmaceutically acceptable salts thereof.

However, the technical challenges of the present invention are not limited to those mentioned above, and other challenges not mentioned will be apparent to those skilled in the art from the following description.

### [Technical solution]

To fulfill the above purposes, the present invention provides a compound represented by the following formula 1, or a pharmaceutically acceptable salt thereof: 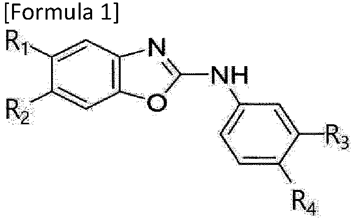
(In Formula 1 above,
R₁ and R₂ may be -H, halogen, or linear or branched C₁₋₆ alkyl, respectively,
R₃ and R₄ may be -H or halogen, respectively).

In one preferred embodiment of the present invention, the compound represented by Formula 1 above may be a compound represented by Formula 1-1 below:

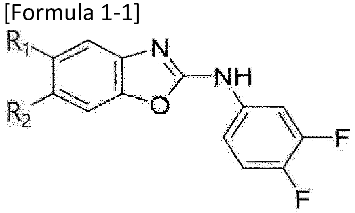

(In Formula 1-1 above, R₁ and R₂ may be -H, halogen, or linear or branched C₁₋₆ alkyl, respectively).

In another preferred embodiment of the present invention, the compound represented by Formula 1-1 may be any one or more selected from the group consisting of:
a compound represented by the following formula 1-1A: 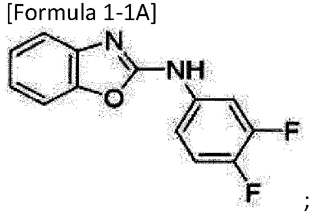
a compound represented by the following formula 1-1B: 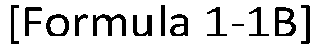 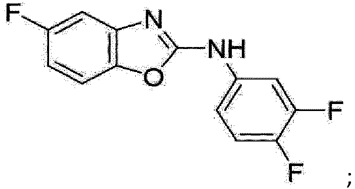
a compound represented by the following formula 1-1C: 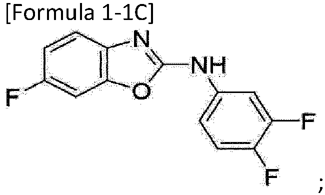 and
a compound represented by the following chemical formula 1-1D:

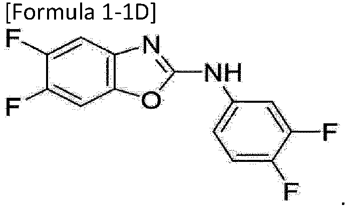

In another preferred embodiment of the present invention, the compound can increase the expression of the Klotho gene.

In another preferred embodiment of the present invention, the compounds can protect retinal pigment epithelial cells from oxidative stress.

### [Advantageous Effects]

The compounds according to the present invention are effective in enhancing the expression of the Klotho gene, a gene associated with aging, as well as having a protective effect against oxidative stress in retinal pigment epithelial cells (RPE), and are therefore useful as a pharmaceutical composition, or as a health functional food composition for the prevention or treatment of macular degeneration.

### [Description of Drawings]

Figure 1 shows the stability of KS compounds according to Example 1 of the present invention confirmed by MS/MS analysis: KS101 (compound of Formula 1-1A), KS102 (compound of Formula 1-1B), KS103 (compound of Formula 1-1C), KS104 (compound of Formula 1-1D).
Figure 2 shows the result of PCR confirmation of the degree of expression of the klotho gene by KS compound according to Example 1 of the present invention.
Figure 3 shows the cytotoxicity of KS compounds according to Example 1 of the present invention.
Figure 4 shows the results of the change in the amount of intracellular Reactive Oxygen Species (ROS) after treatment of renal tubule epithelial cells (RPTECs) with KS compounds of Example 1 of the present invention, respectively.
Figures 5 and 6 show the results of identifying changes in the expression of oxidative stress-related genes by KS compound of Example 1 of the present invention.
Figure 7 shows the results of the cytoprotective effect of KS compounds of Example 1 of the present invention against oxidative stress.

### [Mode of the Invention]

The present invention will now be described in detail.

### Compounds that induce expression of the anti-aging gene KLOTHO

The present invention relates to a compound represented by the following formula 1, or pharmaceutically acceptable salts thereof:

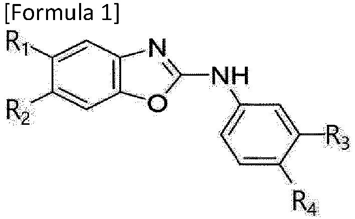

(In Formula 1 above, R₁ and R₂ may be -H, halogen, or linear or branched C₁₋₆ alkyl, respectively, and R₃ and R₄ may be -H or halogen, respectively).

Preferably, the compound represented by Formula 1 above may be the compound represented by Formula 1-1 below:

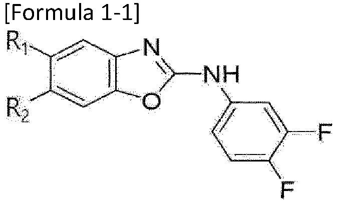

(In Formula 1-1 above, R₁ and R₂ may be -H, halogen, or linear or branched C₁₋₆ alkyl, respectively).

More preferably, the compound represented by Formula 1-1 above may be any one or more selected from the group consisting of:
a compound represented by the following formula 1-1A: 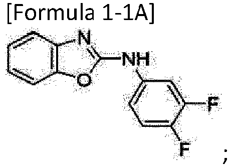
a compound represented by the following formula 1-1B: 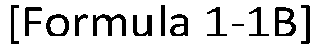 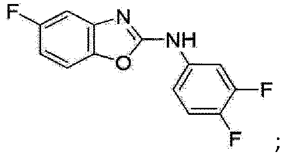
a compound represented by the following formula 1-1C: 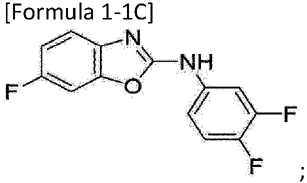 and
a compound represented by the following chemical formula 1-1D:

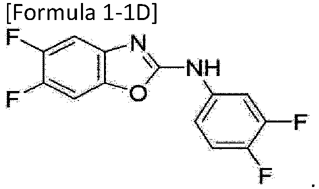

The above compounds of the present invention can be used in the form of pharmaceutically acceptable salts, and as salts, acid adducts formed by pharmaceutically acceptable free acids are useful. The expression pharmaceutically acceptable salt means any organic or inorganic adduct salt of the basic compound of Formula 1 at a concentration that is relatively non-toxic and harmless to the patient, such that side effects attributable to the salt do not detract from the beneficial effects of the basic compound of Formula 1. These salts may be inorganic or organic, with inorganic acids including hydrochloric acid, bromic acid, nitric acid, sulfuric acid, perchloric acid, phosphoric acid, and phosphoric acid, and organic acids including citric acid, acetic acid, lactic acid, maleic acid, maleic acid, fumaric acid, gluconic acid, and methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, or malonic acid. The salts also include alkali metal salts (sodium salts, potassium salts, etc.) and alkali earth metal salts (calcium salts, magnesium salts, etc.). For example, the acid sulfate salts include acetate, aspartate, benzate, besylate, bicarbonate/carbonate, bisulfate/sulfate, borate, campsylate, citrate, edicylate, esylate, formate, fumarate, gluceptate, gluconate, Glucuronate, Hexafluorophosphate, Hybenzite, Hydrochloride/Chloride, Hydrobromide/Bromide, Hydroiodide/lodide, Isethionate, Lactate, Malate, Malyate, Malonate, Mesylate, Methylsulfate, Naphthylate, 2-naphthylate, Nicotinate, Nitrate, Orotate, Oxalate, Palmitate, Pamoate, Phosphate/Hydrogen Phosphate/Dihydrogen Phosphate, Saccharate, Stearate, Succinate, Tartrate, Tosylate, Trifluoracetate, aluminum, alginine, benzathine, calcium, choline, diethylamine, dioleamine, glycine, lysine, magnesium, meglumine, oleamine, potassium, sodium, tromethamine, zinc salts, of which hydrochloride or trifluoroacetate is preferred.

Furthermore, the compounds of the present invention may include not only pharmaceutically acceptable salts, but also all salts, isomers, hydrates and solvates that can be prepared by conventional methods.

The adduct salts according to the invention can be prepared in a conventional manner, for example by dissolving the compound in a water-miscible organic solvent, such as acetone, methanol, ethanol, or acetonitrile, adding an excess of organic acid or an acidic aqueous solution of an inorganic acid, and then precipitating or crystallizing. The mixture is then evaporated of the solvent or excess acid and dried to give the adduct salt, or the precipitated salt is prepared by suction filtration.

The compounds of the present invention can increase the expression of the Klotho gene.

Furthermore, oxidative stress experiments using retinal pigment epithelium confirmed that the compounds of the present invention protect cells from oxidative stress, and thus the compounds of the present invention can be useful as preventive or therapeutic agents for macular degeneration diseases.

### Pharmaceutical composition for the prevention or treatment of macular degeneration

The present invention relates to a pharmaceutical composition for the prevention or treatment of macular degeneration, comprising a compound represented by the following formula 1, or a pharmaceutically acceptable salt thereof, as an active ingredient:

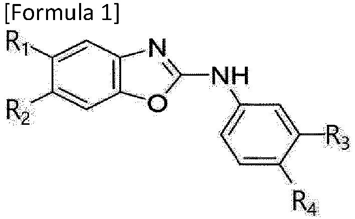

(In Formula 1 above, R₁ and R₂ may be -H, halogen, or linear or branched C₁₋₆ alkyl, respectively, and R₃ and R₄ may be -H or halogen, respectively).

Preferably, the compound represented by Formula 1 above may be the compound represented by Formula 1-1 below:

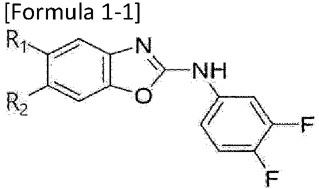

(In Formula 1-1 above, R₁ and R₂ may be -H, halogen, or linear or branched C₁₋₆ alkyl, respectively).

More preferably, the compound represented by Formula 1-1 above may be any one or more selected from the group consisting of:
a compound represented by the following formula 1-1A: 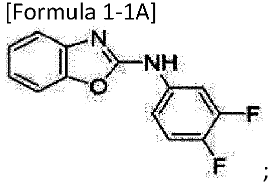
a compound represented by the following formula 1-1B: 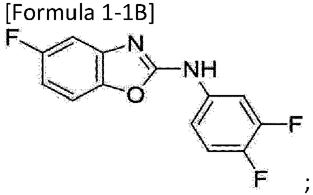
a compound represented by the following formula 1-1C: 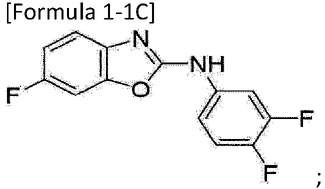 and

A compound represented by the following chemical formula 1-1D:

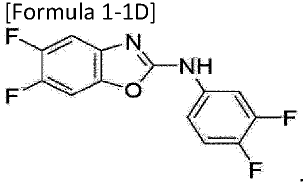

The macular degeneration may be age-related macular degeneration.

The composition of the present invention can increase the expression of the Klotho gene.

Furthermore, the composition of the present invention can protect retinal pigment epithelial cells from oxidative stress.

The compounds of the invention can be administered in a variety of oral and parenteral formulations for clinical administration, and when formulated, they are prepared using commonly used diluents or excipients such as fillers, extenders, binders, wetting agents, disintegrants, and surfactants.

Solid preparations for oral administration include tablets, pills, powders, granules, capsules, troches, and the like, which are prepared by mixing one or more compounds of the invention with at least one excipient, for example, starch, calcium carbonate, sucrose, lactose, or gelatin. In addition to simple excipients, lubricants such as magnesium stearate talc are also used. Liquid preparations for oral administration include suspensions, inclusions, emulsions, or syrups, which may contain a variety of excipients, such as wetting agents, sweeteners, flavors, and preservatives, in addition to the commonly used simple diluents of water and liquid paraffin.

Formulations for parenteral administration include sterile aqueous solutions, nonaqueous solutions, suspensions, emulsions, lyophilizates, suppositories, and the like. Nonaqueous solvents and suspensions may include propylene glycol, polyethylene glycol, vegetable oils such as olive oil, and injectable esters such as ethylolate. As a base for the suppository, witepsol, macrogol, tween 61, cacao paper, laurin paper, glycerol, gelatin, etc. may be used.

Furthermore, the effective dosage of the compounds of the invention in humans may vary depending on the age, weight, gender, dosage form, health status and disease severity of the patient, and is generally about 0.001-100 mg/kg/day, preferably 0.01-35 mg/kg/day. Based on an adult patient weighing 70 kg, the dosage is typically 0.07-7,000 mg/day, preferably 0.7-2,500 mg/day, and may be divided into one to several daily doses spaced at regular intervals as determined by the physician or pharmacist.

### Food Composition for the Prevention or Amelioration of Macular Degeneration

At another point of consistency, the present invention relates to a food composition or a health functional food composition for the prevention or amelioration of macular degeneration, comprising as an active ingredient a compound represented by Formula 1 or a pharmaceutically acceptable salt thereof.

The composition of the present invention can increase the expression of the Klotho gene.

Furthermore, the composition of the present invention can protect retinal pigment epithelial cells from oxidative stress.

The macular degeneration may be age-related macular degeneration.

There is no particular limitation on the type of food, but examples of food to which the composition of the present invention can be added include meat, sausages, bread, chocolate, candy, snacks, confectionery, pizza, ramen, other noodles, gum, and ice cream. It includes dairy products, various soups, beverages, tea, drinks, alcoholic beverages, vitamin complexes, etc., and includes all health foods in the conventional sense.

The health functional food composition containing the active substances according to the invention can be added to food as such or used in combination with other foods or food components, and can be used according to conventional methods as appropriate. The amount of the active substance mixed may be suitably determined according to the purpose of its use (preventive or ameliorative). In general, the amount of the composition in a food or dietary supplement may be added from 0.1 to 90 parts by weight of the total weight of the food (based on a total of 100 parts by weight). However, in the case of long-term consumption for the purpose of health maintenance or health regulation, the amount may be below the above range, and the active substance may be used in amounts above the above range without any safety concerns.

The food and/or health functional food compositions of the present invention are not particularly restricted in terms of other ingredients other than containing the active ingredient of the invention as an essential ingredient in the proportions indicated, and may contain various flavoring agents or natural carbohydrates as additional ingredients, such as conventional beverages. Examples of the natural carbohydrates described above are monosaccharides, e.g., glucose, fructose, etc.; disaccharides, e.g., maltose, sucrose, etc.; and polysaccharides, e.g., dextrins, cyclodextrins, etc.; and sugar alcohols, e.g., xylitol, sorbitol, erythritol, etc. As flavoring agents other than those described above, natural flavoring agents (such as thaumatin, stevia extracts (such as rebaudioside A, glycyrrhizin, etc.) and synthetic flavoring agents (such as saccharin, aspartame, etc.) can be advantageously used. The proportion of the natural carbohydrates is typically from about 1 to 20 g, preferably from about 5 to 12 g, per 100 g of the food or health functional food composition of the invention.

In addition to the above, the food and/or health functional food composition containing the active substances of the present invention may contain various nutrients, vitamins, minerals (electrolytes), flavoring agents such as synthetic and natural flavoring agents, coloring agents and intermediates (cheese, chocolate, etc.), pectic acid and its salts, alginic acid and its salts, organic acids, protective colloidal thickeners, pH regulators, stabilizers, preservatives, glycerin, alcohol, carbonation agents used in carbonated beverages, etc. In addition, the food and/or health functional food composition of the present invention may contain natural fruit juices and pulp for the preparation of fruit juice beverages and vegetable beverages.

These ingredients may be used independently or in combination. The proportions of these additives are not critical, but are typically selected in the range of 0.1 to about 20 parts by weight per 100 parts by weight of the food and/or health functional food composition containing the active substance of the invention.

Hereinafter, the present invention will be described in more detail with reference to the following embodiments. However, the following embodiments are only illustrative of the present invention, and the content of the present invention is not limited by the following embodiments.

### Example 1. Synthesis of Compounds

### 1-1: Synthesizing KS101 Compound

### Step 1: Preparation of 1-(3,4-difluorophenyl)-3-(2-hydroxyphenyl)thiourea (FCCS-19025-2-1)

Under Ar gas atmosphere, 2-Aminophenol (150 mg, 1.37 mmol) was dissolved in methanol (8 mL), then 3,4-difluorophenyl isothiocyanate (224 µl, 1.65 mmol) was slowly added and the mixture was stirred at room temperature for 13 hours. After confirming the termination of the reaction by thin layer chromatography (TLC), methanol was removed under reduced pressure. The reaction mixture was purified by chromatography (Silica-gel column chromatography) (20% Acetone / n-hexane) and 1-(3,4-difluorophenyl)-3-(2-hydroxyphenyl)thiourea (FCCS-19025-2-1) was obtained as a pale yellow solid (354 mg, 92%).

¹H-NMR (400 MHz, MeOH-d₄) *δ* 7.62 (d, *J* = 8.0 Hz, 1H), 7.55 (ddd, *J*= 2.4 Hz, 1H), 7.25-7.13 (m, 2H), 7.11-7.05 (m, 1H), 6.92-6.82 (m, 2H); ESI-(+) 281.3 [M+H]⁺.

### Step 2: Preparation of N-(3,4-difluorophenyl)benzo[d]oxazol-2-amine (FCCS-19025)

Under Ar gas atmosphere, FCCS-19025-2-1 (224 mg, 0.80 mmol) obtained in step 1 above, and Potassium superoxide (KO₂) (284 mg, 4.00 mmol) were dissolved in acetonitrile (MeCN) (25 mL) and stirred at room temperature for 14 hours. After confirming the termination of the reaction by thin layer chromatography (TLC), the acetonitrile (MeCN) was removed under reduced pressure. The reaction mixture was purified by chromatography (Silica-gel column chromatography) (10 to 20% Ethyl acetate/n-hexane) and the target compound, N-(3,4-difluorophenyl)benzo[d]oxazol-2-amine (FCCS-19025), was obtained as a white solid (160 mg, 82%).

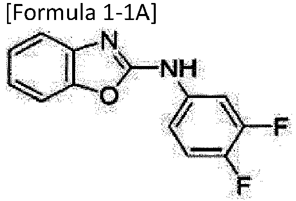

¹H-NMR (400 MHz, MeOH-d₄) *δ* 7.82 (ddd, J= 2.8 Hz, 1H), 7.42 (d, *J* = 7.6 Hz, 1H), 7.36 (d, J = 8.0 Hz, 1H), 7.34-7.29 (m, 1H), 7.28-7.18 (m, 2H), 7.16-7.10 (m, 1H); ESI-(+) 247.2 [M+H]⁺.

### 1-2: Synthesizing KS102 Compound

A mixture of 2-aminophenol (1.0 equiv.) and 3,4-difluorophenyl isothiocyanate (1.2 equiv.) in THF (2 mL/mmol) was stirred at room temperature. After stirring for 3 hours, ferric chloride (FeCl₃.6H₂O) was added to the above solution and the reaction mixture was stirred at 90 °C for 12 hours. The reaction mixture was cooled to room temperature and extracted with ethyl acetate (EtOAc). The combined organic layers were dried over magnesium sulfate (MgSO₄) and concentrated in vacuo. The residue was triturated with dichloromethane (CH₂Cl₂), filtered and washed with dichloromethane, and the desired product (3,4-difluorophenyl isothiocyanate) was obtained as a solid.

Then, the above 3,4-difluorophenyl isothiocyanate (210 mg, 1.23 mmol) and 2-amino-4-fluorophenol (130 mg, 1.02 mmol) were reacted, and KS102 compound (N-(3,4-difluorophenyl)-5-fluorobenzo[d]oxazol-2-amine), represented by Formula 1-1B below, was obtained as a light brown solid (195 mg, 72%).

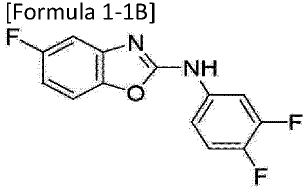

m.p. 222.9-223.9 °C; R_{f} 0.40 (EtOAc/n-hexane = 1:3); HPLC R_{T} 5.15 min (purity: 99.7%);¹ H-NMR (DMSO-d₆, 800 MHz) δ 6.97 (ddd, J = 9.8, 8.7, 2.6 Hz, 1H), 7.36 (dd, J = 9.0, 2.6 Hz, 1H), 7.43-7.48 (m, 2H), 7.52 (dd, J = 8.7, 4.4 Hz, 1H), 7.91 (ddd, J = 12.8, 7.1, 2.3 Hz, 1H), 11.0 (s, 1H);¹³ C-NMR (DMSO-d₆, 200 MHz) 159.4 (J_{CF} = 234.9 Hz), 159.1, 149.2 (J_{CF} = 241.9, 13.0 Hz), 144.7 (J_{CF} = 239.0, 12.4 Hz), 143.4, 143.2 (J_{CF} = 13.7 Hz), 135.5 (J_{CF} = 9.2, 2.2 Hz), 117.8 (J_{CF} = 17.9 Hz), 114.1 (J_{CF} = 5.8, 3.1 Hz), 109.5 (J_{CF} = 10.3 Hz), 108.5 (J_{CF} = 25.6 Hz), 106.7 (J_{CF} = 22.1 Hz), 103.9 (J_{CF} = 26.3 Hz) ppm, LR-MS (FAB) m/z 265 (M+H)⁺; HR-MS (FAB) calcd for C₁₃H₈F₃N₂O (M+H)⁺ 265.0589, found 265.0588.

### 1-3: Synthesizing KS103 Compound

The 3,4-difluorophenyl isothiocyanate (210 mg, 1.23 mmol) obtained in Example 1-2 and 2-amino-5-fluorophenol (130 mg, 1.02 mmol) were reacted, and KS103 compound (N-(3,4-difluorophenyl)-6-fluorobenzo[d]oxazol-2-amine), represented by Formula 1-1C below, was obtained as an ivory colored solid (203 mg, 75%). 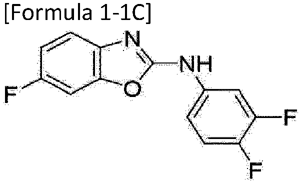 m.p. 231.7-232.6 °C; R_{f} 0.40 (EtOAc/n-hexane = 1:3); HPLC R_{T} 5.12 min (purity: 98.4%);¹ H-NMR (DMSO-d₆, 800 MHz) δ 7.10 (ddd, J = 10.1, 8.6, 2.5 Hz, 1H), 7.42-7.45 (m, 2H), 7.47 (dd, J = 8.6, 4.9 Hz, 1H), 7.54 (dd, J = 8.4, 2.5 Hz, 1H), 7.91 (ddd, J = 12.6, 7.0, 2.1 Hz, 1H), 10.9 (s, 1H);¹³ C-NMR (DMSO-d₆, 200 MHz) 158.1, 158.0 (J_{CF} = 235.9 Hz), 149.2 (J_{CF} = 241.8, 13.2 Hz), 146.8 (J_{CF} = 15.0 Hz), 144.6 (J_{CF} = 238.8, 12.6 Hz), 138.4. 135.7 (J_{CF} = 7.3 Hz), 117.8 (J_{CF} = 17.9 Hz), 116.8 (J_{CF} = 9.6 Hz), 113.8 (J_{CF} = 5.5, 3.3 Hz), 111.0 (J_{CF} = 23.7 Hz), 106.4 (J_{CF} = 22.1 Hz), 98.0 (J_{CF} = 28.9 Hz) ppm, LR-MS (FAB) m/z 265 (M+H)⁺; HR-MS (FAB) calcd for C₁₃H₈F₃N₂O (M+H)⁺ 265.0589, found 265.0586.

### 1-4: Synthesizing KS104 Compound

The 3,4-difluorophenyl isothiocyanate (212 mg, 1.24 mmol) obtained in Example 1-2 and 2-amino-4,5-difluorophenol (150 mg, 1.03 mmol) were reacted, and KS104 compound (N-(3,4-difluorophenyl)-5,6-difluorobenzo[d]oxazol-2-amine), represented by Formula 1-1D below, was obtained as a light purple solid (187 mg, 64%). 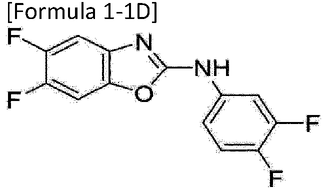 m.p. 242.1-243.2 °C; R_{f} 0.40 (EtOAc/n-hexane = 1:3); HPLC R_{T} 5.44 min (purity: 99.1%);¹ H-NMR (DMSO-d₆, 800 MHz) δ 7.41-7.42 (m, 1H), 7.46 (dd, J = 19.3, 9.0 Hz, 1H), 7.62 (dd, J = 10.5, 7.5 Hz, 1H), 7.83 (dd, J = 9.8, 6.9 Hz, 1H), 7.89 (ddd, J = 13.0, 7.2, 2.5 Hz, 1H), 11.0 (s, 1H);¹³ C-NMR (DMSO-d₆, 200 MHz) 158.9, 149.2 (J_{CF} = 242.0, 13.1 Hz), 147.3 (J_{CF} = 237.0, 13.7 Hz), 145.5 (J_{CF} = 238.0, 14.7 Hz), 144.8 (J_{CF} = 239.0. 13.5 Hz), 142.1 (J_{CF} = 12.6 Hz), 138.0 (J_{CF} = 11.5, 1.5 Hz), 135.4 (J_{CF} = 13.2, 1.8 Hz), 117.8 (J_{CF} = 17.8 Hz), 114.0 ((J_{CF} = 5.7, 3.1 Hz), 106.6 (J_{CF} = 22.1 Hz), 104.9 (J_{CF} = 21.7 Hz), 99.5 (J_{CF} = 24.1 Hz) ppm, LR-MS (FAB) m/z 283 (M+H)⁺ ; HR-MS (FAB) calcd for C₁₃H₇F₄N₂O (M+H)⁺ 283.0495, found 283.0505.

### Experimental Example 1. Stability evaluation of the present compounds

Each KS compound in Example 1 was dissolved in 10 mM DMSO, and tolbutamide, used as a standard, was dissolved in methanol at a concentration of 10 mM.

In a glass test tube (13x100 mm), 0.1 M KH₂PO₄ (147.5 µl), 0.1 M MgCl₂ (25 µl), and 50 µl of human interpersonal microsomes (mixed-gender pool) at a concentration of 0.5 mg/ml purchased from Corning Life Science and each KS compound solution were mixed to a final 1 µM of each KS compound solution, pre-incubated for 3 min in a shaking water bath (37 °C, 40 rpm), and then incubated for 30 min in the presence or absence of 10 mM NADPH, 25 µL.

Aliquots of 50 µl were taken before the reaction and after the reaction, and then the reaction was stopped by adding 2 µl of acetonitrile and vortexed for 1 min. The mixture was centrifuged (4,000 rpm, 4°C) for 20 minutes, and each supernatant was transferred to a new glass test tube and dried for 2 hours using a centrifugal concentrator. After ensuring complete drying, 200 µl of 40% acetonitrile was added to each glass test tube and resuspended by vortexing for 1 minute. The resuspension solution was centrifuged for 10 minutes (4,000 rpm, 4°C) and 150 µl of the supernatant was taken for HPLC-UV analysis. The Agilent 1200 series was used for HPLC-UV analysis and the Agilent 6410 was used for MS/MS analysis. The conditions used for the analysis were as follows:

**[Table 1]**

| Conditions for analysis by HPLC | | | | |
|---|---|---|---|---|
| LC Conditions | | | | |
| Column | Atlantis dC18 100 Å (50 x 2.1 mm i.d., 3.0 µm; Waters, Milford, MA, USA) | | | |
| Column Temp | R.T. (23°C) | | | |
| Mobile phase (A) | 0.1% Formic acid in Water | | | |
| Mobile phase (B) | 0.1% Formic acid in Acetonitrile | | | |
| Autosampler Temp | 10 | | | |
| Injection Volume | 5 µL | | | |
| | | | | |
| Isocratic | Time (min) | Flow rate (µL/min) | A (%) | B (%) |
| | 0 | 200 | 30 | 70 |
| | 3 | 200 | 30 | 70 |

**[Table 2]**

| Conditions for analysis by LC-MS/MS | | | | | |
|---|---|---|---|---|---|
| MS Conditions | | | | | |
| Ionization mode: | Electrospray | | | | |
| Scan mode: | Multiple Reaction Monitoring (MRM) | | | | |
| | | | | | |

| Analyte | Ion Transition (m/z) | Polarity | Retention Time (min) | Fragmentor (eV) | CE (eV) |
|---|---|---|---|---|---|
| KS101 | 247.2 | Positive | 1.69 | 135 | 35 |
| | 80.0 | | | | |
| KS102 | 265.0 | Positive | 1.79 | 135 | 40 |
| | 98.1 | | | | |
| KS103 | 265.0 | Positive | 1.79 | 135 | 30 |
| | 128.0 | | | | |
| KS104 | 283.3 | Positive | 1.91 | 135 | 40 |
| | 141.0 | | | | |
| IS_Tolbutamide | 271.1 | Positive | 1.23 | 135 | 15 |
| | 155.0 | | | | |
| | | | | | |

| MS parameters | | | | | |
|---|---|---|---|---|---|
| Capillary voltage (V): | 4500 | | | | |
| Drying Gas Temp (°C): | 350 | | | | |
| Neubulizing pressure (psi): | 45 | | | | |
| Drying gas flow (L/min): | 11 | | | | |
| | | | | | |

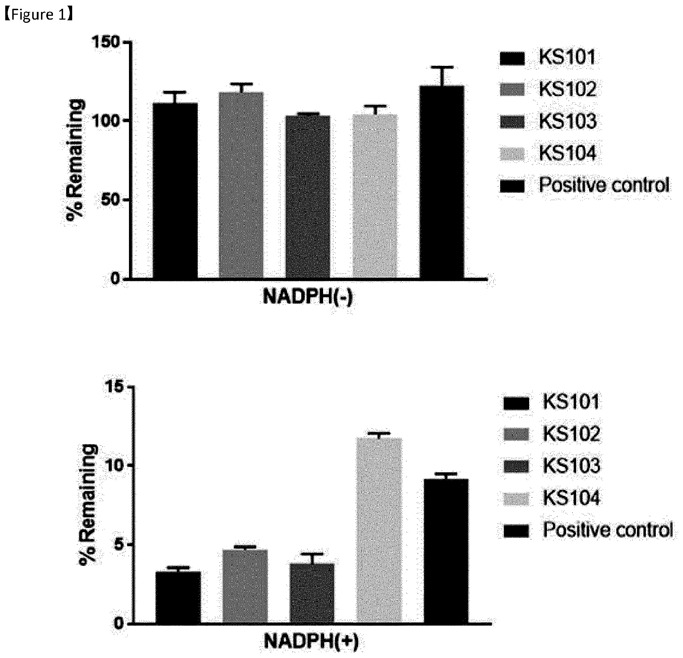

Figure 1 shows the quantitative identification of KS compounds incubated with human liver microsomes by MS/MS analysis. Since the enzymes in the microsomes are NADPH(+)-dependent, the metabolism of the compounds occurred only in reactions in which NADPH(+) was added. The KS compounds showed higher stability than tolbutamide, which was used as a control, and KS104 was found to have the best microsomal stability.

### Experimental Example 2. Comparison of Klotho gene expression effects

Human renal tubular epithelial cells (RPTECs) were treated with each KS compound of Example 1 at different concentrations, and mRNA was collected from the cells 6 hours later, and the mRNA expression of the klotho gene was confirmed by PCR. Renal Proximal Tubular Epithelial Cells (RPTECs) were purchased from Lonza and cultured at 37°C, 5% CO₂ and used in the experiments. Each KS compound was added to the medium of the cells and after 6 hours of exposure, RNA was isolated from the cells and converted to cDNA using reverse transcriptase and amplified by PCR method to determine the expression level. As a control, DMSO was added instead of the compound.

The primers used in the experiment are listed below.
KL-F : GATAGAGAAAAATGGCTTCCCTCC (SEQ ID NO: 1)
KL-R : GGTCGGTAAACTGAGACAGAGTGG (SEQ ID NO: 2)
GAPDH-F : TGACAACTTTGGTATCGTGGAAGG (SEQ ID NO: 3)
GAPDH-R : AGGGATGATGTTCTGGAGAGCC (SEQ ID NO: 4)

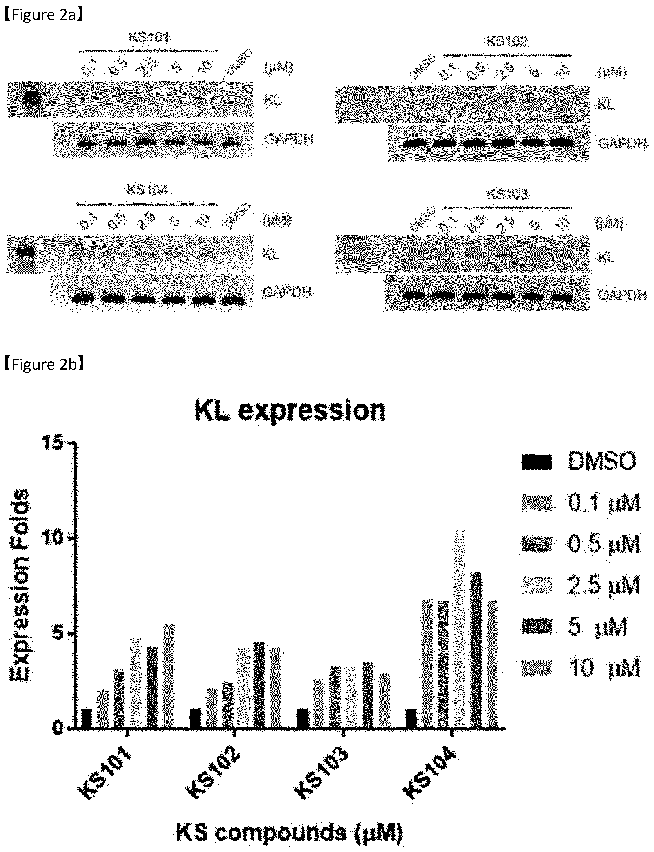

Figure 2 shows the expression of the klotho gene confirmed by PCR. The expression levels of the bands in the gel photographs were quantified using Image J and plotted on a graph. In the gel picture, the upper band represents the secreted form of klotho, and the lower band represents the membrane form of klotho. The quantitative graph shows the sum of the two bands. It was confirmed that KS104 showed the best expression effect and more than doubled the ability to express the klotho gene compared to KS101 as well as other fluorine-bound compounds.

### Experimental Example 3. Cytotoxicity evaluation

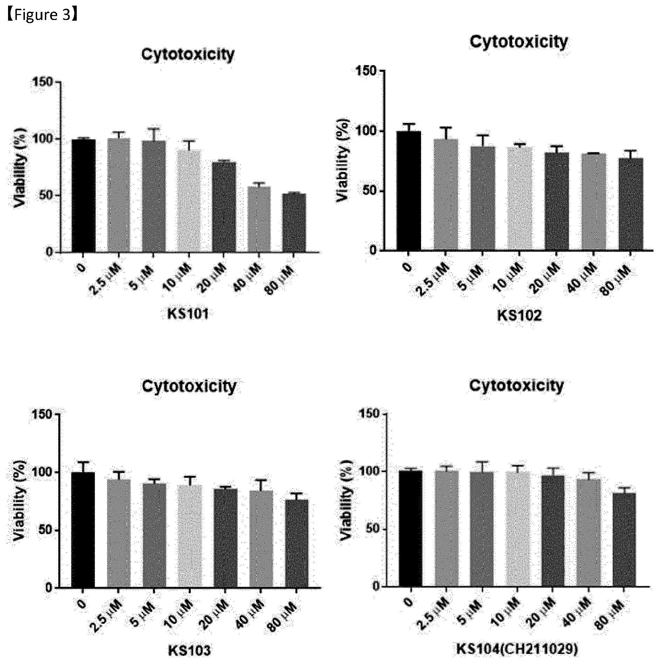

Human retinal epithelial cells (ARPE) in culture were treated with KS compounds of Example 1 at different concentrations and incubated for 24 hours, followed by a 1-hour reaction with EX-cytox from GOGEN, and the change in absorbance was measured. A decrease in absorbance indicates that the cells are toxic and broken. As shown in Figure 3, KS104 was found to have the lowest cytotoxicity.

### Experimental Example 4. Analysis of intracellular reactive oxygen species (ROS)

Renal tubule epithelial cells (RPTECs) were treated with each KS compound of Example 1 to determine the changes in the amount of intracellular reactive oxygen species (ROS).

RPTEC cells in culture were incubated with 2.5 µM of each KS compound for 6 h. The amount of ROS present in the cells was measured by adding Reactive Oxygen Detection Reagents (Invitrogen) and incubating for 1 h. The change in fluorescence was measured using a spectrophotometer.

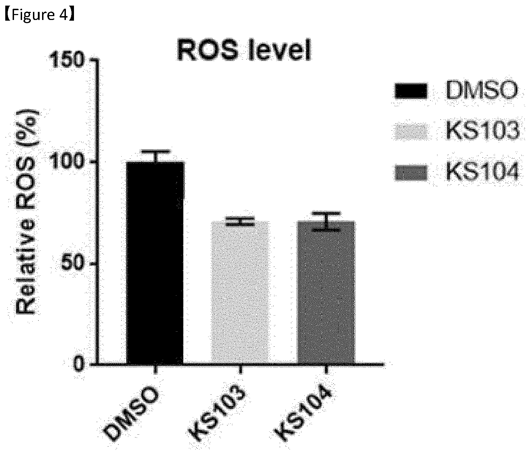

Figure 4 is a graph showing the amount of ROS remaining in RPTEC cells. It was confirmed that the amount of ROS in the cells treated with KS103 and KS104 compounds was reduced by about 30% compared to the cells treated with solvent (DMSO) alone.

### Experimental Example 5. Confirmation of expression changes in oxidative stress-related genes

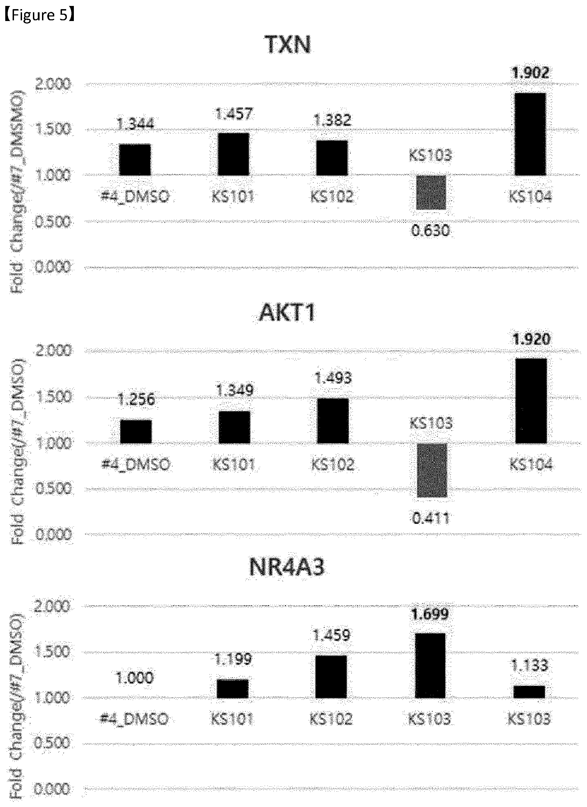

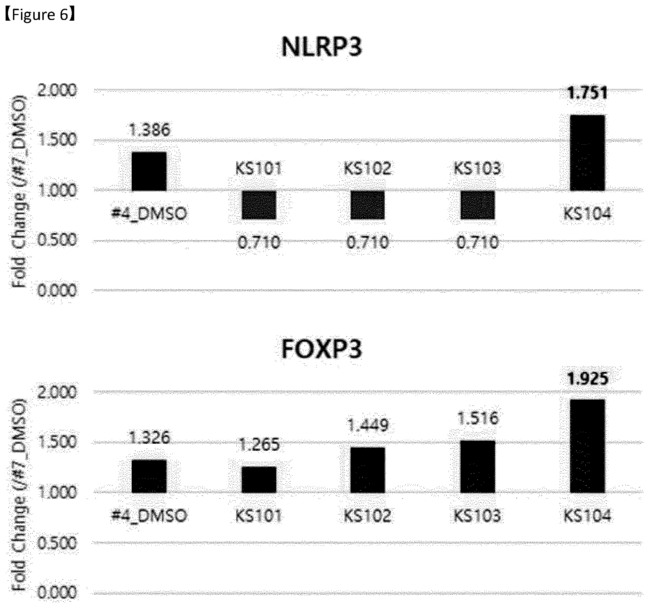

2.5 µM of each KS compound of Example 1 was added to relatively aged 7th passage (#7) renal tubular epithelial cells (RPTEC) cells, cultured for 6 hours, mRNA was collected, and total gene expression was analyzed using a next generation sequencer (NGS). To confirm changes in gene expression due to aging, through gene expression comparison with relatively young 4-passage (#4) RPTEC cells, genes with increased expression in young cells (TXN, AKT1, NR4A3, NLRP3, FOXP3 genes) were confirmed. The expression levels of these genes in young cells were compared to the expression change values caused by treatment with the compounds of Example 1 (senescent cells were treated with the compounds of Example 1, respectively). As a result, it was confirmed that when aged cells were treated with the KS104 compound, the expression of the corresponding gene was induced to a greater extent than in young cells (Figure 5, Figure 6).

### Experimental Example 6. Protective Effect on Retinal Pigment Epithelial Cells

To determine the preventive or therapeutic effect of each KS compound of Example 1 on macular degeneration, the protection of retinal pigment epithelial cells against oxidative stress was determined.

First, human retinal pigment epithelial cells were tested using retinal pigment epithelial cells (ARPE) purchased from ATCC, USA. Equal numbers of 10,000 ARPE cells were added to each well, and after 18 hours of incubation, the cells were replaced with DMEM F-12 medium without serum (FBS).

Each KS compound was added at a concentration of 2.5 µM, and the cells were treated with buthionine sulfoximine (BSO) at a concentration of 50 mM for 6 hours to induce oxidative stress. To measure the viability of these cells, Cyto X (Cell viability assay kit) was added and the time-varying absorbance was measured at 450 nm.

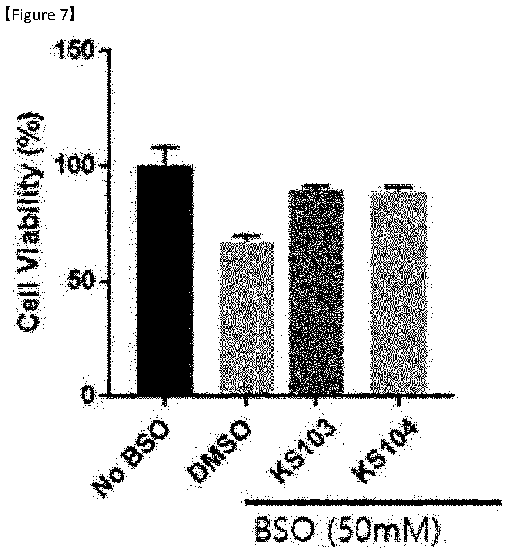

As a result, it was confirmed that both KS103 and KS104 compounds inhibited oxidative stress-induced cell death (Figure 7).

## Claims

1. A compound represented by the following formula 1-1D, or a pharmaceutically acceptable salt thereof:

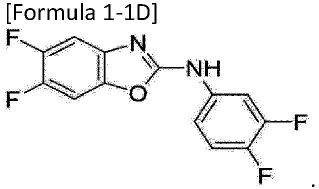

2. A compound represented by the following formula 1-1C, or a pharmaceutically acceptable salt thereof:

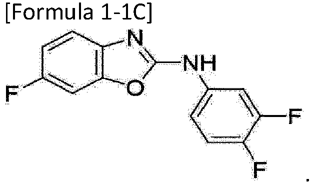

3. The compound or pharmaceutically acceptable salt thereof according to claim 1 or claim 2, wherein the compound increases the expression of the Klotho gene.

4. The compound or pharmaceutically acceptable salt thereof according to claim 1 or claim 2, wherein the compound protects retinal pigment epithelial cells from oxidative stress.

5. The compound or pharmaceutically acceptable salt thereof according to claim 1 or claim 2, wherein the compound is used in a pharmaceutical composition for the prevention or treatment of macular degeneration.

6. The compound or pharmaceutically acceptable salt thereof according to claim 1 or claim 2, wherein the compound is used in a food composition for preventing or ameliorating macular degeneration.
